# EUROPEAN PATENT SPECIFICATION

(11) **EP 0 893 109 B1**
(45) Date of publication and mention of the grant of the patent: **02.03.2005**
(21) Application number: 98305900.7
(22) Date of filing: 23.07.1998
(51) Int. Cl.: A61F 2/08, A61B 17/064, A61B 17/17, A61B 17/88, A61B 17/92

(54) **Apparatus for tibial fixation of soft tissue**
Vorrichtung zur tibialen Befestigung von Weichgewebe
Appareil pour la fixation de tissu mou dans le tibia

(30) Priority: 23.07.1997 US 900602
(43) Date of publication of application: 27.01.1999
(60) Divisional application: 04027643.8
(73) Proprietor: Arthrotek, Inc., Warsaw, Indiana 46580 (US)
(72) Inventor: Boucher, James A., Warsaw, Indiana 46580 (US); Howell, Stephen M., Elk Grove, California 95758 (US); Marcinek, James, Warsaw, Indiana 46580 (US)
(74) Representative: Shaya, Darrin Maurice

(56) References cited:
- EP-A- 0 042 657
- EP-A- 0 330 328
- EP-A- 0 358 372
- EP-A- 0 630 613
- WO-A-98/22047
- FR-A- 2 702 646
- US-A- 4 592 346
- US-A- 4 988 351

## Description

This invention relates generally to an apparatus for use in orthopedic surgery and, more particularly, to an apparatus and method for tibial fixation of a soft tissue graft through a tibial tunnel.

Ligaments are strong fibrous connective soft tissue which connect the articular ends of bones to bind them together and to facilitate or limit motion. Injuries to ligaments are common, and patients who are physically active are generally more susceptible to such ligament injuries. The anterior cruciate ligament (ACL) of the knee joint is a ligament frequently injured by such patients. Such injuries cause instability in the knee joint which, when left untreated, may lead to degenerative arthritis. Because of this condition, ACL reconstruction may be required. Generally during ACL reconstruction, a substitute soft tissue ligament or graft is attached to the femur and/or tibia to facilitate regrowth and permanent attachment.

One method of performing this reconstruction involves the use of a section of bone-patellar tendon-bone as a graft. A ligament tunnel is bored into both the femur and the tibia and the bone-patellar tendon-bone graft is centered between the tunnel. The bone portions of the graft are then each secured within the respective tunnel by tightening an interference screw in each tunnel between the bone graft and the side of the tunnel.

However, use of such a technique presents several disadvantages. For example, the graft may be inadvertently cut or frayed by the sharp edges of the interference screw during insertion of the screw and subsequent to fixation. Moreover, if the interference screw or the bone graft is slightly oversized versus the size of the tunnel, the interference screw may cause too much force to be exerted on the bone graft portion as the interference screw is tightened. This may subsequently cause the bone graft portion to be damaged and not usable. In addition, it is often difficult to accurately gauge the length of the bone-patellar tendon-bone graft in relation to the ligament tunnels such that the bone graft portions may not seat appropriately within the tunnels or be properly tensioned.

Another method for performing this reconstruction involves the use of only a soft tissue ligament graft. Such a graft is generally taken from the hamstring ligament, specifically, the semitendinosus and gracilis ligaments or tendons. Such grafts are generally fed through the ligament tunnel and secured outside the tunnel. The graft is generally secured by a non-endoscopic means of stapling or screwing the graft onto the outside surface of the tibia and/or femur.

However, this method of securing the soft tissue graft also exhibits disadvantages. For example, since the various staple or screw and washer assemblies in existence are positioned on the outside of the bone surface or extend beyond the bone surface, such components are more easily noticed by the patient and in some instances may cause patient discomfort. In addition, because of the discomfort, it may be required to perform subsequent surgery to remove the staple or screw and washer assembly once the graft has permanently attached to the bone, thereby subjecting the patient to a second surgery, as well as increasing overall surgical costs. The staple or screw and washer assembly are also not substantially resistant to slippage and do not provide stiff securement. In other words, the graft may permanently slip under the securement of the staple or screw and washer assembly thereby providing a non-optimum tension on the graft. Securement at the anchoring point may be resilient such that if the graft utilizes sutures in combination with the staple or screw washer assembly, the anchoring point may stretch under stress and resiliently return, thereby also providing non-optimum tensioning or stiffness for the graft.

Another method for securing the soft tissue ligament graft within a femoral tunnel is set forth in U.S. Patent No. 5,431,651. This reference uses a cleated washer which engages the soft tissue graft within the femoral tunnel by use of a transverse cannulated set screw. The cleated washer is drawn into the femoral tunnel by use of a suture coupled to the washer and pulled through the cannulated set screw. Once in position adjacent to the set screw, the set screw engages the cleated washer against the soft tissue ligament and the wall of the tunnel.

However, this method of securing a soft tissue graft within a femoral tunnel also exhibits many disadvantages. For example, such a procedure will generally require more surgical time since it includes the added steps of passing a suture through a cannulated set screw and down the femoral tunnel, as well as attaching it to the cleated washer itself. This also makes it extremely difficult to properly align the cleated washer since the cleated washer must be pulled through and aligned using a flexible non-rigid suture. Additionally, it may be difficult to maintain the location of the cleated washer as the set screw is engaged against the washer since the suture does not rigidly hold or maintain the position of the cleated washer. Finally, by drawing the cleated washer up through the femoral tunnel, a larger femoral tunnel may be required and the spikes on the cleated washer may cut or fray the soft tissue graft as it is passed through the femoral tunnel.

What is needed then is an apparatus for tibial fixation of a soft tissue graft which does not suffer from the above-identified disadvantages. This in turn, will reduce the possibility for damaging the soft tissue graft; reduce the possibility for requiring a new graft from being harvested; provide for endoscopic securement of a soft tissue graft without damaging the graft; reduce or eliminate potential patient discomfort; provide endoscopic fixation which is flush to the bone surface; reduce or eliminate the need for a subsequent surgery to remove fixation components after the graft has been permanently attached to the bone; increased fixation strength; provide less pretensioning of the graft to restore knee stability, thereby not over-constraining the knee after setting the tension on the graft; reduce or eliminate the potential for slippage of the soft tissue graft; and increase stiffness and mechanical behaviour of the soft tissue graft. It is, therefore, an object of the present invention to provide such an apparatus for tibial fixation of a soft tissue graft.

We acknowledge the disclosure in US-A-4592346 of apparatus having the pre-characterizing features of Claim 1 below. US-A-4592346 discloses a rectangular surgical staple with longer spikes for engaging bone and shorter ones for penetrating ligament.

The present invention provides fixation apparatus as defined in Claim 1.

The apparatus may be used for tibial fixation of a soft tissue graft. The apparatus flushly secures the soft tissue graft within a tunnel formed in the tibia. This is basically achieved by utilizing an apparatus which does not extend beyond the tibia upon securing the graft within a tibial tunnel.

Due to claim 1, the apparatus for fixation of a soft tissue graft includes a body having a first side and a second side. A first plurality of spikes extend from the second side of the body each having a first length. A second plurality of spikes extend from the second side of the body each having a second length. The first length is longer than the second length such that the first plurality of spikes are operable to engage the bone without the second plurality of spikes substantially engaging the soft tissue graft to permit proper tensioning of the soft tissue graft.

In a preferred embodiment, the body includes a substantially cylindrical sidewall and a substantially planar relief formed into a portion of the cylindrical sidewall. The substantially planar relief is operable to permit the body to be flushly received within the counterbore and permit the soft tissue graft to exit the counterbore without substantially binding on the cylindrical sidewall and the counterbore.

Use of the present invention provides an apparatus for tibial fixation of a soft tissue graft. As a result, the aforementioned disadvantages associated with the currently available methods and techniques for fixation of soft tissue grafts have been substantially reduced or eliminated.

Still other advantages of the present invention will become apparent to those skilled in the art after reading the following specification by reference to the drawings in which:
FIG. 1 is a side view of an apparatus for tibial fixation of a soft tissue graft according to the teachings of a first preferred embodiment present in the invention;
FIG. 2 is a top view of the tibial fixation apparatus of Fig. 1;
FIG. 3 is a side cross-sectional view of the tibial fixation apparatus of Fig. 1 taken along line 3-3 of Fig. 2;
FIG. 4 is a top view of a counterbore drill guide used in preparing a counterbore to nestingly receive the tibial fixation apparatus of Fig. 1;
FIGS. 5A-5E illustrates a first preferred method for attaching a soft tissue graft using the tibial fixation apparatus according to the teachings of the first preferred embodiment of the present invention;
FIG. 6A-6E illustrates a second preferred method for attaching a soft tissue graft using the tibial fixation apparatus according to the teachings of the first preferred embodiment of the present invention.

The following description of the preferred embodiments concerning an apparatus for tibial fixation of soft tissue grafts are merely exemplary in nature and are in no way intended to limit the invention or its application or uses. Moreover, while the present invention is described in detail below with reference to tibial fixation of soft tissue grafts through a tibial tunnel, it will be appreciated by those skilled in the art that the present invention is clearly not limited to fixation through a tibial tunnel and may be utilized to secure soft tissue grafts, tendons, ligaments, etc. through various other tunnels, bores or adjacent to a bone.

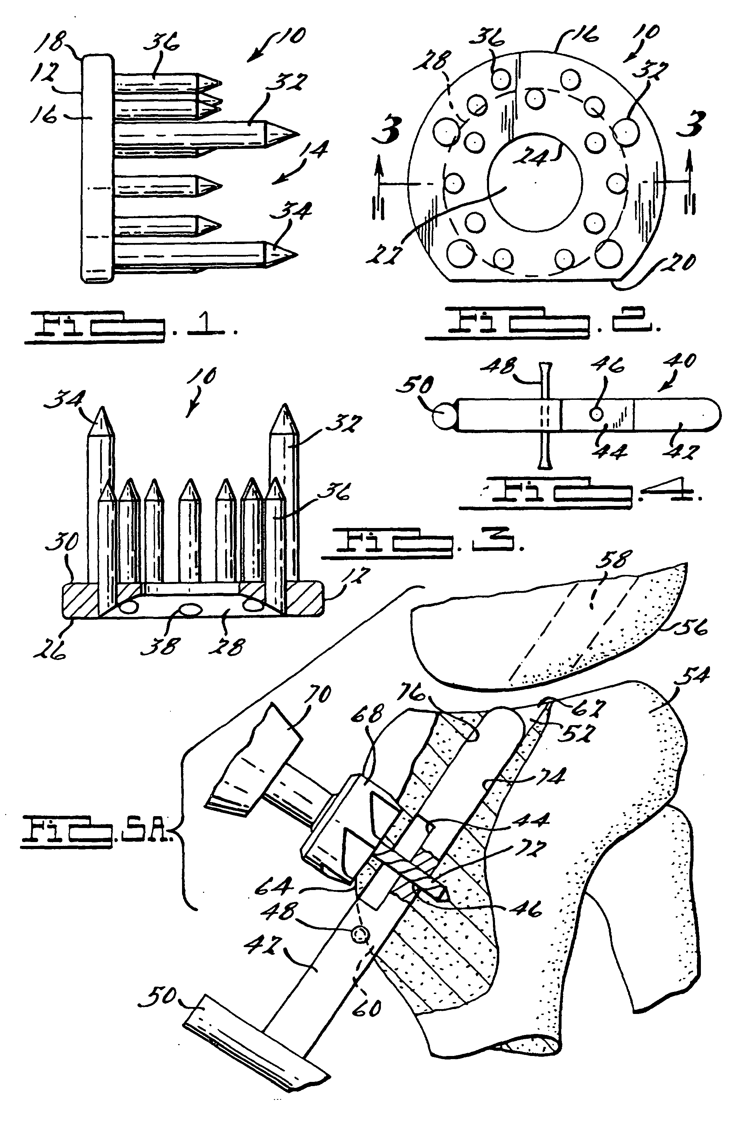

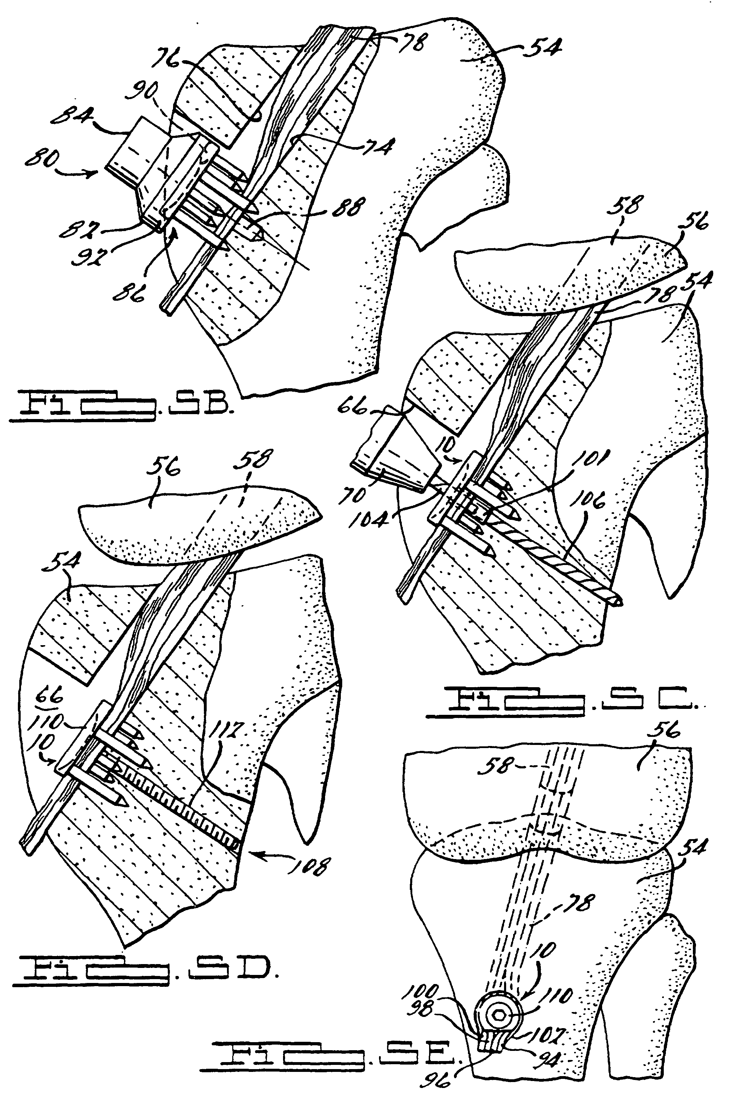

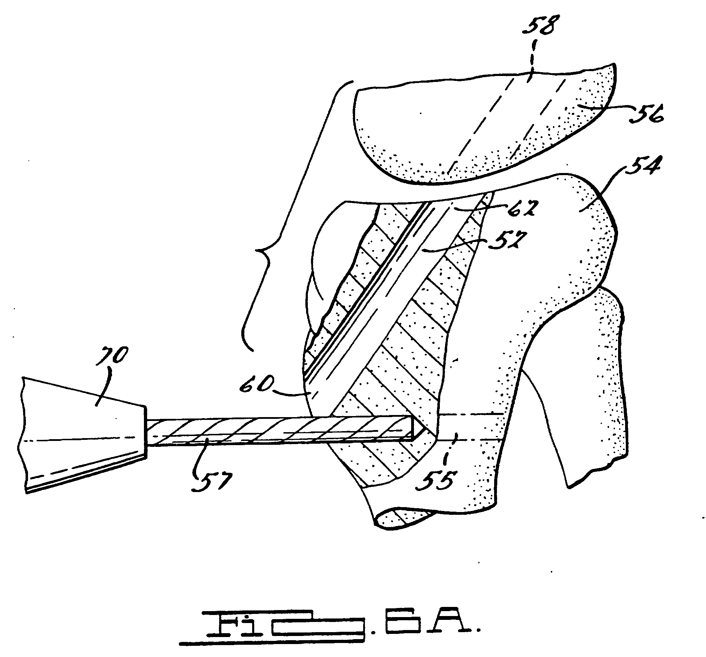

Referring to Figs. 1-3, an apparatus 10 for tibial fixation of a soft tissue graft according to the teachings of a first preferred embodiment of the present invention is shown. The apparatus or washer 10 includes a cylindrical body 12 having a diameter of about 16 millimeters and a plurality of spikes 14. The cylindrical body 12 includes a substantially cylindrical sidewall 16 having beveled or rounded edges 18 and a substantially flat planar relief face 20 formed into a portion of the cylindrical sidewall 16. The body 12 further defines a concentric internal bore 22 having an internal sidewall 24 with a diameter of about 0,59 cm (0.234 inches) which is adapted to receive a compression bone screw, further discussed herein. A first side or top 26 of the body 12 includes a concentric counterbore 28 operable to flushly receive a head of the compression screw. A second side or bottom 30 of the body 12 is substantially planar and has the plurality of spikes 14 extending out from the second side 30.

The plurality of spikes 14 includes a first plurality or four cylindrical guide spikes 32 each having a length from the top 26 of the body 12 to the tip 34 of the spikes 32 of about 1,32 cm (0.52 inches) and a cylindrical diameter of about 0,16 cm (.062 inches). The four spikes 32 are positioned concentrically about the body 12 at a radius of about 0,63 cm (0.25 inches) from the center of the body 12. The plurality of spikes 14 further includes a second plurality or thirteen cylindrical engagement spikes 36 each having a length from the top 26 of the body 12 to the tip 34 of the spikes 36 of about 0,86 cm (0.34 inches) and a cylindrical diameter of about 0,12 cm (.047 inches). The cylindrical spikes 32 and 36 each include the pointed end 34 operable to engage and penetrate cancellous and cortical bone. The engagement spikes 36 are positioned between the guide spikes 32 and the internal bore 22 in such a manner that the engagement spikes 36 are able to penetrate and secure the soft tissue graft at multiple sites, further discussed herein.

The apparatus or washer 10 is preferably made from a suitable biocompatible material such as titanium, stainless steel, titanium alloy, cobalt-chrome-molybdenum alloy, polymer, resorbable polymer, etc. The apparatus 10 preferably consists of an assembly of separate spikes 14 which are welded to the body 12, via welds 38. Alternatively, the apparatus 10 may be cast or machined to the required shape and size.

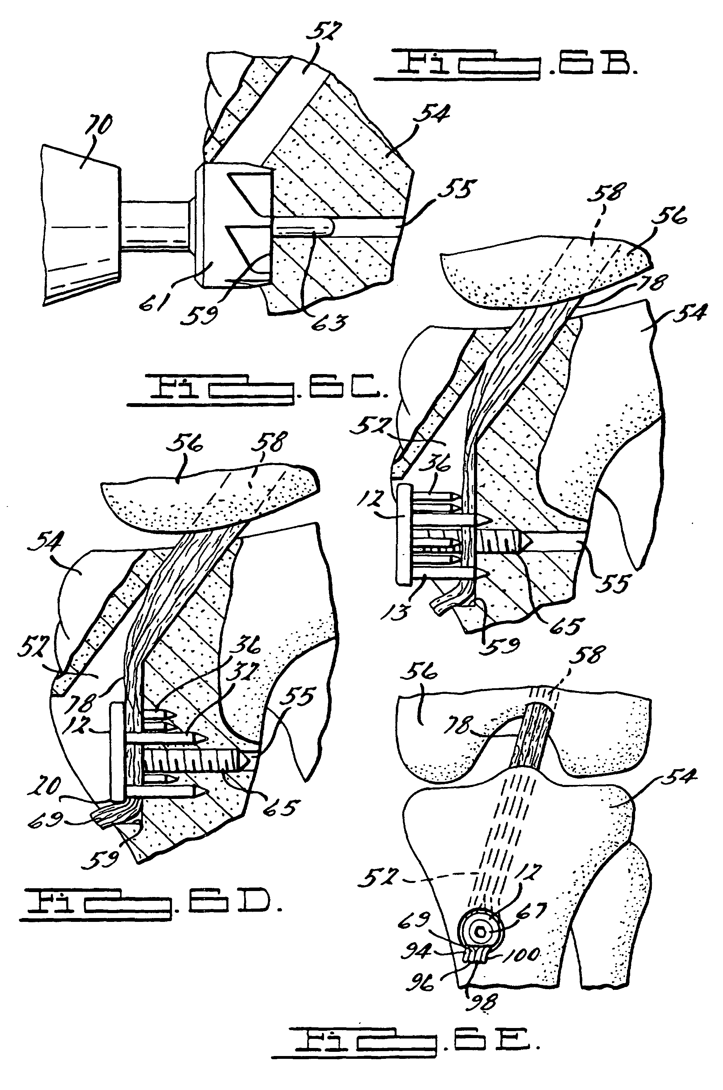

Referring to Figure 4, a counterbore drill guide 40 is shown for use in preparation of a substantially perpendicular counterbore relative to a tibial tunnel formed in a tibia, further discussed herein. The counterbore drill guide 40 includes a substantially cylindrical body 42 defining a planar notched region 44 having a center aperture 46 for receipt of a counterbore drill bit. Positioned adjacent to the notched region 44 is a positioning bar 48 passing medially through the body 42. The positioning bar 48 is slidably engaged with the body 42 and is utilized to engage the medial cortex of the tibia to provide a predetermined insertion length of the counterbore drill guide 40 into the tibial tunnel. Set back from the positioning bar 48 is a T-shaped handle 50 which can be utilized by the surgeon to hold the counterbore drill guide 40 during counterbore drilling. The body 42 of the counterbore drill guide 40 is about 7 millimeters in diameter, with the notched region 44 beginning at about 20 millimeters from a distal end of the drill guide 40. The notched region 44 is about 17 millimeters wide having about a 3.5 millimeter diameter centering hole 46. The positioning bar 48 is set back about 10 millimeters from the center of the centering hole 46, thereby providing a predetermined insertion length within the tibial tunnel of about 38.5 millimeters. The counterbore drill guide 40 is preferably made from stainless steel or other suitable material.

Turning to Figures 5A-5E, a first preferred method for tibial fixation of a soft tissue graft will now be described. Initially, soft tissue grafts are harvested for use in an intrarticular cruciate ligament (ACL) reconstruction. The semitendinosus and gracilis tendons from their respective hamstring muscles are generally used and harvested from the patient or by way of donor tendons using techniques well known in the art. Alternatively, synthetic grafts may also be employed. The harvested grafts will generally consist of two tubular grafts which are subsequently looped at their mid-section to form a four bundle graft. The four bundle graft should be sized to pass without friction through a tibial and femoral tunnel.

Once the grafts have been harvested and prepared using known techniques, tunnel or hole placement is then performed. A tibial tunnel or bore 52 is drilled through the tibia 54 and into the femur 56 creating a femoral tunnel 58. The tibial tunnel 52 will typically have a diameter between about 7 to 13 millimeters, preferably 8-9 millimeters, and is bored utilizing a drill bit and a driver. The tibial tunnel 52 exits at about the center of the tibial plateau and enters the tibia 54 at about 50 millimeters from the top of the tibial plateau medial to the tibial tubercle or at the medial cortex. Since the tibial tunnel 52 angles through the tibia 54, it creates an elliptical entrance opening 60 and an elliptical exit opening 62. The drill bit utilized to bore the tibial tunnel 52 also generally bores the femoral tunnel 58 in the femur 56 by continuing to extend the drill bit through the tibial tunnel 52 and into the femur 56. The tibial tunnel 52 and the femoral tunnel 58 are bored using techniques well known in the art which may include the use of alignment or drill guide mechanisms in combination with a drill bit and driver.

Once the tibial tunnel 52 is bored through the tibia 54, the body 42 of the counterbore drill guide 40 is axially slid into the entrance opening 60 of the tibial tunnel 52. The counterbore drill guide 40 is advanced into the tibial tunnel 52 until the positioning bar 48 is flush against the medial cortex of the tibia 54. This will align the centering hole 46 adjacent to an anterior edge 64 of the entrance opening 60 of the tibial tunnel 52.

With the counterbore drill guide 40 properly positioned within the tibial tunnel 52 and held by the handle 50, a counterbore 66 is formed substantially perpendicular to the tibial tunnel 52 using a counterbore bit 68 and a drive mechanism 70. The counterbore bit 68 includes a centering nose 72 which rotatably engages the centering hole 46 formed into the counterbore drill guide 40. The centering nose 72 is formed similar to a drill bit so that the centering nose 72 drills into the tibia 54. The counterbore 66 is preferably bored to remove the anterior edge 64 of the entrance opening 60 providing a substantially perpendicular counterbore 66. In other words, the centering nose 72 is directed through the tibia 54 and inserted in the centering hole 46 in the counterbore drill guide 40 such that the counterbore bit 68 is perpendicular to a posterior wall 74 of the tibial tunnel 52. The counterbore bit 68 is advanced to remove the anterior tibial cortex adjacent to an anterior wall 76 until it seats in the notch region 44 in the counterbore drill guide 40. The bone reamings may then be collected and saved for subsequent grafting of the tibial tunnel 52.

Once the counterbore 66 has been formed utilizing the counterbore bit 68, a four bundle graft 78 is first secured within the femoral tunnel 58 of the femur 56 using one of many techniques known in the art. Preferably, the four bundle graft 78 is secured within the femoral tunnel 58 of the femur 56 by means of a bone mulch screw, set forth in "BONE MULCH SCREW ASSEMBLY FOR ENDOSTEAL FIXATION OF SOFT TISSUE GRAFTS AND METHOD FOR USING SAME", filed November 18, 1994 and naming as inventors Stephen M. Howell and Roy C. Wiley, U.S. Serial No. 08/342,324, Notice of Allowance dated February 19, 1997. With the graft 78 secured within the femoral tunnel 58, the graft 78 extends out through the tibial tunnel 52.

The apparatus 10 is then engaged and secured to the tibia 54 by use of an impactor 80 in combination with a mallet. The impactor 80 has a generally cylindrical body 82 which tapers to a striking end 84. The impactor 80 includes a complimentary face 86 having a concentric guide post 88 which is received within the internal bore 22 and an arcuate surface 90 which is received within the counterbore 28. The impactor 80 further includes an edge guide 92 which engages and mates with the planar relief face 20 formed within the apparatus 10. The edge guide 92 enables the surgeon to properly rotatably align the apparatus 10 by simply rotating the impactor 80 within the counterbore 66 and engaging the planer face 20 with the edge guide 92.

The graft 78 is oriented such that two grafts 94 and 96 of the four bundle graft 78 pass along a first side of the guide post 88 and two grafts 98 and 100 pass along a second side the guide post 88 such that each pair of grafts are positioned or guided between the guide post 88 and the longer guide spikes 32. The apparatus 10 is initially partially engaged with only the guide spikes 32 penetrating cancellous and cortical bone of the tibia 54 and the relief face 20 directed toward the entrance opening 60. With the guide spikes 32 engaging the tibia 54, the four bundle graft 78 is appropriately tensioned by pulling the four bundle graft 78 under the engagement spikes 36 within the counterbore 66 and out of an area 102 defined by the relief 20 and the counterbore 66 without binding on the sidewall 16 or the counterbore 66. The two stage spikes 14 having the first plurality of spikes 32 of a first length and the second plurality of spikes 36 having a second shorter length enables the apparatus 10 to be initially secured to the tibia 54 with only the spikes 32, while allowing proper tensioning and guiding of the four bundle graft 78.

The four bundle graft 78 is properly tensioned generally by means of pulling on the ends of the graft 78 extending out of the area 102 under the relief 20 manually or with a tensioning device with the knee in full extension. A preferred tensioning device is set forth in U.S. Patent No. 5,507,750 entitled, "METHOD AND APPARATUS FOR TENSIONING GRAFTS AND LIGAMENTS". Once the proper tension is achieved, the apparatus 10 is fully seated or nested within the counterbore 66 by use of the impactor 80 and a mallet, as shown clearly in Fig. 5C. Upon seating the apparatus 10, the engagement spikes 36 penetrate the two grafts 94 and 96 on the first side of the guide post 88 at multiple sites and the two grafts 98 and 100 on the second side of the guide post 88 to maintain proper tensioning of the four bundle graft 78. The apparatus or washer 10 thus seats or nests flush within the counterbore 66 thereby eliminating any objects extending out beyond the tibia 54. The relief 20 is also used to eliminate any portion of the apparatus 10 from extending out beyond the tibia 54.

With the apparatus 10 fully nested within the counterbore 66 or posterior wall 74 of the tibia tunnel 52, a drill guide 101 is inserted into the internal bore 22 of the apparatus 10 to maintain the separation of the four bundle graft 78. The drill guide 101 is advanced between the two grafts 94 and 96 on the first side of the guide post 88 and the two grafts 98 and 100 on the second side of the guide post 88 so that it is flush against the posterior wall 74 of the tibial tunnel 52. A 3.5 millimeter drill bit 104 attached to the driver 70 is then utilized to drill a bore 106 through the tibia 54 to the posterior cortex of the tibia 54. Once the bore 106 is drilled through the tibia 54, the depth of the bore 106 is measured and the posterior cortex region is tapped using an appropriate tap.

With the bore 106 formed and tapped, a low profile compression screw 108 is inserted into the internal bore 22 and screwed into the bore 106 in the tibia 54 to threadably secure the compression screw 108 within the bore 106 and complete the fixation of the apparatus 10 within the counterbore 66. The compression screw 108 includes a head 110 which is flushly received within the counterbore 28, a threaded section 112, and a cylindrical non-threaded section 114 passing through the body 12 of the apparatus 10. Once the compression screw 108 has been fully secured, the ends of the grafts 94-100 may be trimmed back within the area 102. The apparatus 10 provides a substantially stiff and slippage free anchoring for the graft 78.

Turning to Figures 6A-6E, a second preferred method for tibial fixation of a soft tissue graft will now be described. In this regard, like reference members will be used to reference to like structures. Once the grafts have been harvested and prepared using the known techniques, tunnel or hole placement is again performed. The tibial tunnel or bore 52 is drilled through the tibia 54 and into the femur 56 creating a femoral tunnel 58. The tibial tunnel 52 will typically have a diameter between about 7 to 13 millimeters, preferably 8-9 millimeters, and is bored utilizing a drill bit and a driver. The tibial tunnel 52 exits at about the center of the tibial plateau and enters the tibia 54 at about 50 millimeters from the top of the tibial plateau medial to the tibial tubercle or at the medial cortex. Since the tibial tunnel 52 angles through the tibia 54, it creates an elliptical entrance opening 60 and an elliptical exit opening 62. The drill bit utilized to bore the tibial tunnel 52 also generally bores the femoral tunnel 58 in the femur 56 by continuing to extend the drill bit through the tibial tunnel 52 and into the femur 56. The tibial tunnel 52 and the femoral tunnel 58 are bored using techniques well known in the art which may include the use of alignment or drill guide mechanisms in combination with a drill bit and driver.

Once the tibial tunnel 52 is bored through the tibia 54, a pilot hole 55 is bored straight through the tibia 54 parallel with the tibia plateau. Specifically, the pilot hole 55 is started at the intersection point at the bottom of the entrance 60 and is drilled from the anterior medial to posterior lateral side utilizing a drill bit 57 driven by the drive mechanism 70. The pilot hole 55 is preferably about 2 to 3 millimeters in diameter.

Once the pilot hole 55 has been formed or bored, a counterbore 59 is formed at the anterior medial side of the tibia 54 using a counterbore bit 61 and the drive mechanism 70. The counterbore bit 61 includes a pilot nose 63 which engages the pilot hole 55 to accurately align the counterbore bit 61 concentric with the pilot hole 55 and relative to the entrance opening 60. The counterbore 59 is preferably bored to a depth of about 6 to 7 millimeters which intersects with the tibial tunnel 52 or entrance opening 60.

Once the counterbore 59 has been formed utilizing the counterbore bit 61, the four bundle graft 78 is first secured within the femoral tunnel 58 of the femur 56 using one of the many techniques known in the art. Here again, the four bundle graft 78 is preferably secured within the femoral tunnel 58 of the femur 56 by means of the bone mulch screw, set forth in "BONE MULCH SCREW ASSEMBLY FOR ENDOSTEAL FIXATION OF SOFT TISSUE GRAFTS AND METHOD FOR USING SAME", filed November 18, 1994 and naming as inventors Stephen M. Howell and Roy C. Wiley, U.S. Serial No. 08/342,324, Notice of Allowance dated February 19, 1997. With the graft 78 secured within the femoral tunnel 58, the graft 78 extends out through the tibial tunnel 52.

The apparatus 10 is then engaged to the tibia 54 by means of a bone screw 65 having a head 67 and the spikes 32. The graft 78 is oriented such that the two grafts 94 and 96 of the four bundle graft 78 pass along a first side of the bone screw 65 and two grafts 98 and 100 pass along a second side the bone screw 65 such that the pair of grafts are positioned or guided between the bone screw 65 and the longer guide spikes 32. The apparatus 10 is initially partially engaged with only the guide spikes 32 penetrating cancellous and cortical bone of the tibia 54 and the bone screw 65 partially set.

With the guide spikes 32 engaging the tibia 54, the four bundle graft 78 is appropriately tensioned by pulling the four bundle graft 78 under the engagement spikes 32 along the counterbore 59 and out of an area 69 defined by the relief 20 and the counterbore 59 without binding on the sidewall 16 or the counterbore 59. The two stage spikes 14 having the first plurality of spikes 32 of a first length and the second plurality of spikes 36 having a second shorter length enables the apparatus 10 to be initially secured to the tibia 54 with only the spike 32 and bone screw 65, while allowing tensioning and guiding of the four bundle graft 78.

With the four bundle graft 78 properly tensioned, generally by means of pulling on the ends of the graft 78 extending out of the area 69 under the relief 20, the bone screw 65 is further turned to fully seat the apparatus or washer 10 flush within the counterbore 59, as shown clearly in Fig. 6D. Upon turning the bone screw 65, the engagement spikes 36 penetrate the two grafts 94 and 96 on the first side of the bone screw 65 and the two grafts 98 and 100 on the second side of the bone screw 65 to maintain proper tensioning of the four bundle graft 78. the apparatus or washer 10 thus seats flush within the counterbore 59, thereby eliminating any objects extending out beyond the tibia 54. Once the bone screw 65 has been fully secured, the ends of the grafts 94-100 may be trimmed back within the area 64.

## Claims

1. An apparatus for fixation of a soft tissue graft (78) to a bone (54), said apparatus comprising a body (12), a first plurality of spikes (32), each of said first plurality of spikes (32) having a first length and a first pointed end (34), and a second plurality of spikes (36), each of said second plurality of spikes (36) having a second length and a second pointed end (34), said first length being longer than said second length and said second plurality of spikes being operable to engage and pierce the soft tissue graft (78) with said second pointed ends (34),
said body (12) has a first side (26) and a second side (30) and said first and second pluralities of spikes extend from said second side (30),
said first plurality of spikes (32) are adapted to engage and pierce the bone (54) with the first pointed ends (34), and
the difference in length of said first and second spikes is such that said first plurality of spikes (32) are able to pierce and engage said bone (54) without said second plurality of spikes (36) substantially engaging the soft tissue graft (78) thereby to permit proper tensioning of the soft tissue graft (78),
**characterised in that** said body (12) is a substantially circular cylinder.

2. The apparatus as defined in Claim 1, **characterised in that** said body includes a substantially cylindrical sidewall (16) and a substantially planar relief (20) formed into a portion of said substantially cylindrical sidewall (16).

3. The apparatus as defined in Claim 2 **characterised in that**, in use, said body (12) is nested within a counterbore (66) and said relief (20) permits said soft tissue graft (78) to exit the counterbore (66) without binding on said substantially cylindrical sidewall (16) and said counterbore (66).

4. The apparatus as defined in claim 1 **characterized in that** said body (12) defines an internal bore (22) and a concentric counterbore (28) formed within the first side (26) of said body (12) operable to receive a screw having a head.

5. The apparatus as defined in any one of the preceding claims **characterised in that** each of said first plurality of spikes (32) has a first diameter and wherein each of said second plurality of spikes (36) has a second diameter, said first diameter being larger than said second diameter.

6. The apparatus as defined in any one of the preceding claims **characterised in that** said first plurality of spikes (32) retains and guides the soft tissue graft (78) under the second plurality of spikes (36) whereby said second plurality of spikes (36) are operable to pierce said soft tissue graft (78) at a plurality of sites,

7. The apparatus as defined in any one of the preceding claims further **characterised by** a counterbore drill guide (40) having a substantially cylindrical body (42) defining a notched region (44) with a centering hole (46) operable to receive a counterbore bit (68).

8. The apparatus as defined in any one of the preceding claims further **characterised by** an impactor (80) having a complimentary face (86) which mates with said first side (26) of said body (12) and including an edge guide (92) operable to engage the or a substantially planar relief (20) formed into a portion of a substantially cylindrical sidewall (16) of said body (12).

9. The apparatus as defined in Claim 8 wherein said impactor further includes a guide post (88) which is operable to be received within the or an internal bore (22) defined by said body (12).

## Patentansprüche

1. Vorrichtung zur Befestigung von Weichgewebe-Transplantat (78) an einem Knochen (54), wobei die Vorrichtung einen Körper (12) enthält, eine Vielzahl von Nägeln (32), wobei die erste Vielzahl von Nägeln (32) eine erste Länge und ein erstes spitzes Ende (34) aufweist, und eine zweite Vielzahl von Nägeln (36), wobei die zweite Vielzahl von Nägeln (36) eine zweite Länge und ein zweites spitzes Ende (34) aufweist, wobei die erste Länge größer ist als die zweite Länge, und die zweite Vielzahl von Nägeln so funktioniert, dass sie das Weichgewebe-Transplantat (78) mit den zweiten spitzen Enden (34) erfasst und aufspießt,
der Körper (12) eine erste Seite (26) und eine zweite Seite (30) aufweist und dass die erste und zweite Vielzahl von Nägeln sich von der zweiten Seite (30) aus erstrecken,
die erste Vielzahl von Nägeln (32) so angepasst sind, dass sie den Knochen (54) mit den ersten spitzen Enden (34) erfassen und aufspießen, und
die Längendifferenz von der ersten und zweiten Vielzahl von Nägeln so angelegt ist, dass die erste Vielzahl von Nägeln (32) in der Lage ist, den Knochen (54) ohne dass die zweite Vielzahl von Nägeln (36) das Weichgewebe-Transplantat (78) im wesentlichen erfasst, wobei dadurch ein gutes Spannen des Weichgewebe-Transplantats (78) ermöglicht wird,
**dadurch gekennzeichnet, dass** es sich bei dem Körper (12) um einen im wesentlichen kreisförmigen Zylinder handelt,

2. Vorrichtung gemäß Definition nach Anspruch 1, **dadurch gekennzeichnet, dass** der Körper eine im wesentlichen zylindrische Seitenwand (16) enthält und ein im wesentlichen ebenes Relief (20), welches als Teil der den, im wesentlichen zylindrischen Seitenwand (16) ausgebildet ist.

3. Vorrichtung gemäß Definition nach Anspruch 2, **dadurch gekennzeichnet, dass** der Körper (12) während des Gebrauchs in einer Gegenbohrung (66) eingesetzt ist und das Relief (20) erlaubt, dass das Weichgewebe-Transplantat (78) die Gegenbohrung (66) verlassen kann, ohne an der im wesentlichen zylindrischen Seitenwand (16) und an der Gegenbohrung (66) hängenzubleiben.

4. Vorrichtung gemäß Definition nach Anspruch 1, **dadurch gekennzeichnet, dass** der Körper (12) während des Gebrauchs eine interne Bohrung (22) und eine konzentrische Gegenbohrung (28) definiert, welche innerhalb der ersten Seite (26) des den Körpers (12) gebildet werden und in der Weise funktionieren, dass sie eine mit einem Kopf versehene Schraube aufnehmen können.

5. Vorrichtung gemäß Definition nach irgend einem der vorausgehenden Ansprüche, **dadurch gekennzeichnet, dass** jede der ersten Vielzahl von Nägeln (32) einen ersten Durchmesser aufweist, und bei der jeder der zweiten Vielzahl von Nägeln (36) einen zweiten Durchmesser aufweist, wobei der erste Durchmesser größer ist als der zweite Durchmesser.

6. Vorrichtung gemäß Definition nach irgend einem der vorausgehenden Ansprüche, **dadurch gekennzeichnet, dass** die erste Vielzahl von Nägeln (32) das Weichgewebe-Transplantat (78) unter der zweiten Vielzahl von Nägeln (36) festhält und führt, wobei die zweite Vielzahl von Nägeln (36) in der Weise funktioniert, dass sie das Weichgewebe-Transplantat (78) an einer Vielzahl von Stellen aufspießen kann.

7. Vorrichtung gemäß Definition nach irgend einem der vorausgehenden Ansprüche, ferner **dadurch gekennzeichnet, dass** eine Bohrbuchse (40) fiir die Gegenbohrung mit einem im wesentlichen zylindrischen Körper (42) vorhanden ist, welcher einen eingekerbten Bereich (44) mit einer Zentrierbohrung (46) definiert, welche in der Weise funktioniert, dass sie eine Gegenbohrungs-Bohrspitze (68) aufnehmen kann.

8. Vorrichtung gemäß Definition nach irgend einem der vorausgehenden Ansprüche, ferner **dadurch gekennzeichnet, dass** ein Stoßelement (80) mit einer zusätzlichen Fläche (86) vorhanden ist, welche mit der ersten Seite (26) des den Körpers (12) zusammenpasst und auch eine Kantenführung (92) enthält, welche in der Weise funktioniert, dass sie das oder ein im wesentlichen ebene(s) Relief (20), welches als Teil einer im wesentlichen zylindrischen Seitenwand (16) des Körpers (12) gebildet wurde erfassen kann.

9. Vorrichtung gemäß Definition nach Anspruch 8, wobei das Stoßelement darüber hinaus einen Führungszylinder (88) enthält, welcher in der Weise funktioniert ist, dass er innerhalb der oder einer durch den Körper (12) definierten Innenbohrung (22) aufgenommen werden kann.

## Revendications

1. Dispositif pour la fixation d'un greffon de tissu mou (78) à un os (54), ledit dispositif comprenant un corps (12), une première pluralité de broches (32), ladite première pluralité de broches (32) ayant une première longueur et une première extrémité en pointe effilée (34), et une seconde pluralité de broches (36), ladite seconde pluralité de broches (36) ayant une seconde longueur et une seconde extrémité en pointe effilée (34), ladite première longueur étant plus longue que ladite seconde longueur et ladite seconde pluralité de broches pouvant être exploitée pour s'agrafer au greffon de tissu mou (78) et le percer au moyen desdites secondes extrémités en pointe (34),
ledit corps (12) présentant une première face (26) et une seconde face (30), et lesdites première et seconde pluralités de broches se prolongeant à partir de ladite seconde face (30),
ladite première pluralité de broches (32) étant adaptée de façon à s'agrafer à l'os (54) et à le percer au moyen desdites premières extrémités en pointe effilée (34), et
la différence de longueur entre lesdites premières et secondes broches étant telle que ladite première pluralité de broches (32) est capable de percer l'os (54) et de s'y agrafer sans qu'un agrafage important de ladite seconde pluralité de broches (36) au greffon de tissu mou (78) n'ait lieu, assurant ainsi un tensionnage approprié du greffon de tissu mou (78),
**caractérisé par le fait que** ledit corps (12) correspond à un cylindre essentiellement circulaire.

2. Dispositif selon la revendication 1, **caractérisé par le fait que** ledit corps comporte une paroi latérale essentiellement cylindrique (16) et une surface évidée essentiellement plane (20) formée dans une portion de ladite paroi latérale essentiellement cylindrique (16).

3. Dispositif selon la revendication 2, **caractérisé par le fait qu'**en cours d'utilisation, ledit corps (12) est emboîté dans un épaulement (66) et ladite surface évidée (20) permet la sortie du greffon de tissu mou (78) de l'épaulement (66) sans adhésion à ladite paroi latérale essentiellement cylindrique (16) ni audit épaulement (66)

4. Dispositif selon la revendication 1, **caractérisé par le fait que** ledit corps (12) définit un vide d'alésage interne (22) et un épaulement concentrique (28) formés dans ladite première face (26) dudit corps (12) et pouvant recevoir une vis à fente.

5. Dispositif selon l'une quelconque des revendications précédentes, **caractérisé par le fait que** chacune des broches de ladite première pluralité de broches (32) présente un premier diamètre et chacune des broches de ladite seconde pluralité de broches (36) un second diamètre, ledit premier diamètre étant plus élevé que ledit second diamètre.

6. Dispositif selon l'une quelconque des revendications précédentes, **caractérisé par le fait que** ladite première pluralité de broches (32) maintient et guide le greffon de tissu mou (78) sous la seconde pluralité de broches (36), ladite seconde pluralité de broches (36) pouvant ainsi être exploitée pour percer ledit greffon de tissu mou (78) à une pluralité de sites.

7. Dispositif selon l'une quelconque des revendications précédentes, également **caractérisé par le fait qu'**un guide d'alésage de l'épaulement (40) présentant un corps essentiellement cylindrique (42) définit une région entaillée (44) qui comporte un trou de centrage (46) pouvant recevoir une mèche-fraise (68).

8. Dispositif selon l'une quelconque des revendications précédentes, également **caractérisé par le fait qu'**il comprend un impacteur (80) présentant une surface complémentaire (86) adaptée à ladite première face (26) dudit corps (12) et un guide margeur (92) permettant le positionnement d'une ou de ladite surface évidée essentiellement plane (20) formée dans une portion de ladite paroi latérale essentiellement cylindrique (16) dudit corps (12).

9. Dispositif selon la revendication 8, dans lequel ledit impacteur comprend également une tige de guidage (88) pouvant être insérée dans un ou ledit trou interne (22) défini par ledit corps (12).
